# EUROPEAN PATENT SPECIFICATION

(11) **EP 2 749 301 B1**
(45) Date of publication and mention of the grant of the patent: **07.08.2019**
(21) Application number: 13199727.2
(22) Date of filing: 27.12.2013
(51) Int. Cl.: A61L 27/44, A61L 27/46, A61L 27/48, A61L 27/56, C08L 67/04, A61L 27/50

(54) **Composites for osteosynthesis**
Verbundwerkstoffe für Osteosynthese
Composites pour ostéosynthèse

(30) Priority: 28.12.2012 US 201261746990 P; 13.03.2013 US 201313799537
(43) Date of publication of application: 02.07.2014
(73) Proprietor: DePuy Synthes Products, Inc., Raynham, MA 02767 (US)
(72) Inventor: Beck, Stefan, 4436 Oberdorf (CH); Niederberger, Lorenz, 4436 Oberdorf (CH); Stohler, Nico, 4436 Oberdorf (CH)
(74) Representative: Carpmaels & Ransford LLP

(56) References cited:
- EP-A1- 1 891 984
- US-A1- 2007 191 963
- US-A1- 2008 069 852
- US-A1- 2011 076 762

## Description

### FIELD OF THE INVENTION

The present invention generally relates to biocompatible composites suitable for implantation into a patient for osteosynthesis. In further embodiments, the present invention relates to methods for making biocompatible composites for osteosynthesis.

### BACKGROUND

Improvements to devices useful for repairing bone defects and/or fractures are highly sought after in the medical community. Among the sought after properties are improvements to ease of implantation, operating room efficiencies, biological acceptance by the patient, speed of recovery and treatment success. Improvements to handling and mechanical properties are of particular interest for use in fragility fractures and during repositioning processes. Improvements to treat trauma indications are also of interest including metaphyseal fractures in the proximal humerus, the distal femur and the proximal tibia.
US 2007/191963 A1 relates to an osteoimplant composite comprising a plurality of particles of an inorganic material, a bone substitute material, a bone-derived material, or any combination thereof; and a polymer material with which the particles are combined.
US 2008/069852 A1 relates to porous composites for application to a bone defect site to promote new bone growth comprising a biocompatible polymer and a plurality of particles of bone-derived material, inorganic material, bone substitute material or composite material.
EP 1891984 relates to a cement powder comprising an organic component consisting of one or more biocompatible and bioresorbable polymers and an inorganic component consisting of one or more calcium phosphate compounds.

### BRIEF SUMMARY OF THE INVENTION

The present invention relates to a biocompatible, resorbable composite for osteosynthesis as defined in claim 1. In some embodiments, the polymer matrix comprises from about 5% to about 80% of the weight of the composite. In yet other embodiments, the polymer matrix comprises from about 10% to about 50% of the weight of the composite. In some embodiments, the composite is completely synthetic. In some embodiments, at least the osteoconductive particles and/or the polymer matrix is synthetic. In some embodiments, the chemical additive may or may not be synthetic.

In some embodiments, the osteoconductive particles include or consist of a bioactive glass. In some embodiments, the osteoconductive particles include or consist of a silica-based bioglass. In other embodiments, the osteoconductive particles include or consist of a ceramic material. The ceramic material may be substantially crystalline or semi-crystalline. In certain embodiments, the osteoconductive particles include or consist of a calcium phosphate. For example, in some embodiments, the osteoconductive particles consist essentially of beta-tricalcium phosphate. In some embodiments, the osteoconductive particles include or consist essentially of hydroxyapatite. In some embodiments, the calcium phosphate is crystalline or semi-crystalline. In other embodiments, the calcium phosphate is amorphous. In some embodiments, the osteoconductive particles include a calcium phosphate, a magnesium phosphate, a magnesium sulfate, a silica based bioglass, or mixtures thereof. In further embodiments, the osteoconductive particles can each have a broadest dimension of about 0.5 mm to about 1.4 mm. In some embodiments, the osteoconductive particles can each have a broadest dimension of about 0.5 mm to about 5.6 mm. In some embodiments, the osteoconductive particles can each have a broadest dimension of about 1.4 mm to about 5.6 mm.

In some embodiments, the polymer matrix includes or consists essentially of a poly (hydroxy carboxylic acid) based polymer. In some embodiments, the polymer matrix is a porous polycaprolactone matrix. In some variations of these embodiments, the polymer matrix consists essentially of poly-(ε)-caprolactone. In other embodiments, the polymer matrix does not include polycaprolactone. In some embodiments, the polymer matrix includes a caprolactone copolymer, for example, PLA-CL, PLA-GA-CL, PCL-PEG, or combinations thereof. The polymer matrix includes polymer particles (e.g., polycaprolactone particles) affixed to an external surface of the osteoconductive particles. In some embodiments, the osteoconductive particles are physically connected by the polymer particles. A portion of the external surface of the osteoconductive particles is not covered by the polymer particles. Moreover, in some embodiments, the osteoconductive particles
are in fluid communication with the pores of the polycaprolactone matrix. In some embodiments, the polymer matrix has a porosity of about 20% to about 80%.

In some embodiments, the composite of the present invention is in a substantially pliable state at a temperature from about 40 degrees C to about 90 degrees C, and wherein the composite is in a substantially rigid state at a temperature of about 37 degrees C or less. In further embodiments, the composite is configured to transition from the substantially pliable state to the substantially rigid state without a chemical reaction.

In some embodiments, the chemical additive is configured to create an acidic environment within the polymer matrix when the composite is implanted. In one such embodiment, the chemical additive comprises an acidic compound. For example, the acidic compound may be selected from the group consisting of lactic acid, lactic acid dimer, lactic acid oligomer, capric acid monomer, capric acid dimer, capric acid oligomer, glycolic acid monomer, glycolic acid dimer, glycolic acid oligomer, ascorbic acid, citric acid, fatty acids and their metal salts, and combinations thereof. In further embodiments, the chemical additive is configured to increase the degradation rate of the polymer matrix when the composite is implanted. In an exemplary embodiment where the polymer matrix includes or consists of a polycaprolactone matrix, the chemical additive may be configured to facilitate breakage of ester linkages within the polycaprolactone matrix when the composite is implanted.

In other embodiments, the chemical additive comprises a hydrophilic compound. In some embodiments, the chemical additive is configured to increase the wettability of the polymer matrix. In some embodiments, the chemical additive and/or increasing the wettability of the polymer matrix allows for an enhanced hydrophilic attack of the polymer matrix with body fluids after implantation. In further embodiments, the chemical additive comprises a polymer, such as a polymer selected from the group consisting of polyethylene glycol, polyethylene oxide, polypropylene glycol, poly lactic acid, poly glycolic acid, and copolymers thereof. The chemical additive is preferably not copolymerized with the polymer matrix. In some embodiments, the chemical additive includes a plasticizer and where the polymer matrix includes a polymer that, in the presence of the plasticizer, has handling properties similar to polycaprolactone.

In another embodiment of the invention, a biocompatible, resorbable composite for osteosynthesis includes osteoconductive particles comprising beta-tricalcium phosphate dispersed within a porous polycaprolactone matrix, the polycaprolactone matrix comprising a plurality of fluid passageways that expose at least a portion of a plurality of the osteoconductive particles to an exterior of the polycaprolactone matrix, wherein the osteoconductive particles have a median particle size of about 0.5 mm to about 1.4 mm in a broadest dimension, wherein the osteoconductive particles are about 50% to about 70% by weight of the composite, and wherein the polycaprolactone matrix is about 30% to about 50% by weight of the composite. In one variation of this embodiment, the osteoconductive particles consist of beta-tricalcium phosphate. In a further variation of this embodiment, the osteoconductive particles are about 70% by weight of the composite, and wherein the polycaprolactone matrix is about 30% by weight of the composite. In yet further variations of this embodiment, the osteoconductive particles have a median particle size of about 0.5 mm to about 0.7 mm in a broadest dimension. In some embodiments of this composite, a chemical additive configured to modify one or more properties of the polycaprolactone matrix is included in the composite.

The method for making a composite according to the claims of the present invention includes the steps of: mixing the osteoconductive particles with a polymer material (e.g., polycaprolactone) and the chemical additive to form a mixture, and treating the mixture to bind the osteoconductive particles with the polymer material to create a solid unit, said polymer material forming a polymer matrix. The method according to another embodiment of the invention further includes shaping the mixture using a press or mold. For example, in some embodiments, the mixture is shaped into a disc, cylinder, tablet, wafer, plug, cone, frustum, rod or other desired shape. In yet another embodiment, the method further includes packaging the solid unit.

In some embodiments of the method, the mixing step includes combining the osteoconductive particles with the polymer material in a solvent, and wherein the treating step comprises removing the solvent. In one such embodiment, removing the solvent includes lyophilization and/or evaporation of the solvent. In further embodiments of the invention, the treating step comprises heating the mixture. In some embodiments, the treating step comprises exposing the mixture to microwave radiation.

In other embodiments of the method, the mixing step comprises coating the polymer material onto the osteoconductive particles. For example, in some embodiments, coating the polymer material onto the osteoconductive particles includes spray coating and/or dip coating. The surface of the osteoconductive particles is not entirely coated by the polymer material. In other embodiments, the osteoconductive particles and the polymer material are melt extruded. The mixing step includes mixing the osteoconductive particles with polymer material particles at about room temperature. In other embodiments, the mixing step comprises mixing the osteoconductive particles with the polymer material with a heatable stirring unit. In some embodiments, the polymer material is mixed with the chemical additive prior to mixing with the osteoconductive particles.

### BRIEF DESCRIPTION OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention can be embodied in different forms and thus should not be construed as being limited to the embodiments set forth herein.
FIG. 1A shows a side view of an example composite made in accordance with an embodiment of the present invention;
FIG. 1B shows a top view of the example composite of FIG. 1A.
FIG. 2 is a micrograph image showing details of a composite made in accordance with an embodiment of the invention;
FIG. 3 is a graph showing the results of an eight-week study of various composites made in accordance with a further embodiment of the present invention;
FIGS. 4A-4D show the implantation of an example composite into a bone according to one embodiment of the present invention;
FIGS. 5A-5C show the implantation of a second example composite into a bone according to another embodiment of the present invention; and
FIG. 6 is a radiograph image of the bone shown in FIGS. 5A-5C after implantation of the second example composite.

### DETAILED DESCRIPTION OF THE INVENTION

The present subject matter will now be described more fully hereinafter with reference to the accompanying figures and examples, in which representative embodiments are shown. The present subject matter can, however, be embodied in different forms and should not be construed as limited to the embodiments or examples set forth herein.

The present invention includes biocompatible composites configured for use in osteosynthesis. Composites according to certain embodiments of the present invention are particularly suited for replacing or augmenting bone and/or treating or repairing bone defects. For example, in some embodiments, the composites are useful as fillers for fractures or voids in bone, for repair of metaphyseal or craniomaxillofacial (CMF) defects, and/or for spinal augmentation or fusion. In some embodiments, the composites after implantation are drillable and possess sufficient mechanical strength to support attachment of bone screws or other bone fixation devices. The composites are at least partially resorbable, preferably fully resorbable, such that the composites are eventually replaced by native bone tissue after implantation. In some embodiments, the composites are configured to be fully resorbed in less than twenty-four months after implantation. In some embodiments, the composites are configured to be fully resorbed in less than eighteen months after implantation. In some embodiments, the composites are configured to be fully resorbed in less than twelve months after implantation. In some embodiments, the composites are configured to be fully resorbed within a period from about six months to about eighteen months after implantation. In further variations, the composites are completely synthetic. In some embodiments, synthetic materials are more likely to yield consistent quality and higher quantity over non-synthetic materials.

Moreover, composites according to some embodiments of the present invention are configured to reversibly transition between a substantially rigid state and a substantially pliable state. For example, composite materials may be substantially solid at body temperate but pliable when heated to a temperature above normal body temperature. In some embodiments, the process of transitioning from a substantially solid material to a pliable material is repeatable -for example, up to three times. The composite, in some implementations is both fully resorbable and able to provide support during a reduction procedure. Additionally, in some embodiments, the composite is osteoconductive and machinable (e.g., drillable or tappable).

In some embodiments, the composites in the substantially pliable state can be molded or formed (e.g., by hand) into desired shapes, for example, to match the contours of a patient's bone. The composites in the substantially pliable state may also be manipulated and formed using surgical tools, for example, raspatories, forceps, pliers, curettes, scapels, and/or other instruments. In some embodiments, surgical tools or other devices (e.g., bone splitters) may be inserted or embedded into the composite when the composites are in the substantially in the pliable state. One potential advantage of composites with such a property is the flexibility it affords a surgeon to reduce a fracture and fill a defect before fixation. In preferred embodiments, the composites are configured to transition from the substantially rigid state to the substantially pliable state without the addition of further additives (such as chemical additives) to the composite including, for example, solvents, plasticizers, softeners, or the like. For example, composites containing only bela-tricalcium phosphate as an osteoconductive particulate or granule and a polymer matrix of poly-(ε)-caprolactone (PCL) may provide the desired properties.

Likewise, in some embodiments, the transition from the substantially pliable state and the substantially rigid state occurs without the addition or release of solvents, monomers, or other compounds from the composite. In other preferred embodiments, the transition between the substantially pliable state and the substantially rigid state occurs without the need for any chemical reactions (e.g., polymerization reactions).

In some embodiments, the composites are configured to transition from the substantially rigid state to the substantially pliable state when the composites are heated to a temperature at or above a predetermined temperature such as glass transition temperature (T_{g}) or melting point (Tₘ) and further configured to return to the substantially rigid state when cooled to a transition temperature, e.g., below T_{g} or Tₘ. In some embodiments, a substantially rigid composite may be heated to a temperature above T_{g} or Tₘ by immersing the composite in a hot water bath or exposing the composite to a heat lamp or other radiation source, which can be performed immediately prior to implantation, in order to soften the composite and allow it to be molded or shaped into a desired form. The composite may then be implanted into the patient (e.g., to fill a bone cavity or fracture) and further shaped if desired at the point of implantation. Once the composite is allowed to cool (e.g., to a temperature below T_{g} or Tₘ), the composite returns to the substantially rigid state while maintaining the desired form.

In some embodiments, for example, the composites of the present invention are substantially rigid at temperatures at or below about normal human body temperature (about 37 degrees C) such that the composites become substantially rigid after implantation. In some embodiments, the composites become substantially pliable at a temperature greater than about 37 degrees C, for example, at about 40 degrees C to about 90 degrees C. In some embodiments, composites according to embodiments of the present invention are configured to become substantially pliable at temperatures greater than 50 degrees C, greater than 55 degrees C, greater than 60 degrees C, greater than 65 degrees C, greater than 70 degrees C, or greater than 75 degrees C. In some embodiments, T_{g} or Tₘ of the composites is preferably less than 100 degrees C, less than 95 degrees C, less than 90 degrees C, less than 85 degrees C, less than 80 degrees C, or less than 75 degrees C.

Composites according to some embodiments of the invention have a yield strength of at least or greater than 2 MPa (or about 2 MPa) in the substantially rigid state. In some embodiments, the composites further have a modulus of about 80 MPa to about 440 MPa in the substantially rigid state. In some embodiments, the composites have a modulus of at least 80 MPa in the substantially rigid state. In some embodiments, the composites have a modulus of at least 100 MPa in the substantially rigid state. In some embodiments, the composites have a modulus of at least 200 MPa in the substantially rigid state. In some embodiments, the composites have a modulus of at least 400 MPa in the substantially rigid state. In some embodiments, the composites have a modulus greater than 440 MPa in the substantially rigid state. These and other desirable characteristics may be achieved by composites according to the present invention which includes osteoconductive particles dispersed within a resorbable polymer matrix. A composite according to the present invention includes a plurality of osteoconductive particles and polymer particles that are aggregated to form the composite. The composite includes an aggregate of osteoconductive particles, each of the osteoconductive particles being partially covered by polymer particles. The polymer particles are adhered to the external surface of the osteoconductive particles and may function to bind the osteoconductive particles together. The composite may include for, example, a blend of granules that are cohesive at room temperature. Each of the granules may include one or more osteoconductive particles that are at least partially covered by one or more polymer particles. Without wishing to be bound by any particular theory, the polymer particles bond to the osteoconductive particles are characterized by a cohesiveness at room temperature such that mass of granules form a rigid structural mass. For example, adjacent osteoconductive particles within the composite may be bridged by one or more polymer particles. The external surface of each of the osteoconductive particles is not entirely covered by polymer particles.

The composites may also be friable, according to some embodiments, such that one or more of the granules made up of osteoconductive particles and/or polymer particles can be separated from the composite. In some embodiments, the composites may become friable when the composites are in the substantially pliable state. Under certain circumstances, the friability of the composite may allow for the composite to be readily broken apart, for example, such that a surgeon may reduce the size of a composite to fit a particular application.

The polymer matrix formed from the polymer particles is porous. One benefit of a composite that includes both osteoconductive particles contained within a porous polymer matrix is the accessibility of body fluids to the osteoconductive particles thereby enhancing the bioacceptance of the implant. The polymer matrix includes a plurality of fluid passageways that expose at least a portion of the osteoconductive particles embedded in the polymer matrix to an exterior of the polymer matrix such that, upon implantation into a patient, body fluids can penetrate into the polymer matrix and come into direct contact with at least a portion of the osteoconductive particles. In preferred embodiments, the osteoconductive particles remain at least partially exposed by the fluid passageways before, during, and after transition of the composite from the substantially rigid state to the substantially pliable state and vice versa.

Composites of the present invention additionally include at least one additive incorporated into the polymer matrix which are selected and configured to modify one or more of acidity, degradation rate, melting point, hydrophilicity, and hydrophobicity of the polymer matrix.

The various components of the composites according to embodiments of the present invention are discussed in further detail below. The headings used herein are included solely to make the disclosure easier to understand and should not be considered limiting in any way.

### I. Osteoconductive Particles

As discussed herein, composites according to some embodiments of the present invention include a plurality of osteoconductive particles. In preferred embodiments, the osteoconductive particles include or consist of discrete particles of an osteoconductive material. The discrete particles may be in the form of aggregated materials. As used herein, "osteoconductive" refers to the ability to provide an appropriate scaffold or template for bone formation or growth. For example, in some embodiments, the osteoconductive particles can be used to form an implant that allows the growth of bony tissue into the structure of the implant.

In some embodiments, the osteoconductive particles make up about 20% to about 95% by weight of the composite. In some embodiments, the osteoconductive particles make up about 50% to about 90% by weight of the composite. In some embodiments, the osteoconductive particles make up about 60% to about 80% by weight of the composite. In some embodiments, the osteoconductive particles make up about 70% by weight of the composite. In some embodiments, the osteoconductive particles make up at least 20%, at least 30%, at least 40%, or at least 50% by weight of the composite. In some embodiments, the osteoconductive particles make up greater than 50% by weight of the composite.

Each of the osteoconductive particles may be generally spherical or may be any other geometric shape including, for example, cubic, cylindrical, conic, pyramidal, etc. In other embodiments, the particles may be irregularly shaped or filamentous. Moreover, in certain embodiments, the osteoconductive particles may themselves include porous particles. The osteoconductive material may be supplied in granules, blocks, wedges or other manufactures.

In some embodiments, the osteoconductive particles of the present invention range in size from about 0.5 mm to about 5.6 mm in a broadest dimension (e.g., in diameter for spherical particles). In some embodiments, the osteoconductive particles of the present invention range in size from about 0.5 mm to about 1.4 mm in a broadest dimension. In some embodiments, the osteoconductive particles of the present invention range in size from about 1.4 mm to about 5.6 mm in a broadest dimension. In some embodiments, the osteoconductive particles range in size from about 0.5 mm to about 0.7 mm in a broadest dimension. In other embodiments, the osteoconductive particles average in size from about 0.7 mm to about 1.4 mm in a broadest dimension. In some embodiments, the osteoconductive particles are at least 0.1 mm in a broadest dimension, at least 0.25 mm in a broadest dimension, at least 0.5 mm in a broadest dimension, at least 0.7 mm in a broadest dimension, or at least 1.0 mm in a broadest dimension. In some embodiments, the osteoconductive particles are no more than 10.0 mm in a broadest dimension, no more than 5.6 mm in a broadest dimension, no more than 5.0 mm in a broadest dimension, no more than 2.0 mm in a broadest dimension, no more than 1.4 mm in a broadest dimension, or no more than 0.7 mm in a broadest dimension. In other embodiments, the osteoconductive particles have a broadest dimension at least or greater than 1.4 mm. In some embodiments, the osteoconductive particle size is chosen to manipulate the porosity of the resulting composite. For example, in some embodiments, the osteoconductive particle size is selected to maximize or allow for increased porosity of the resulting composite.

Materials suitable for use in the osteoconductive particles include biocompatible natural materials and synthetic materials, preferably resorbable materials. Examples of natural materials suitable for the osteoconductive particles according to some embodiments include bone-derived materials such as allograft bone and demineralized bone. In other embodiments, the osteoconductive particles are derived entirely from synthetic materials. As noted above, in some embodiments synthetic materials may provide more consistent quality and higher quantity over natural materials and in some embodiments, 100% synthetic materials may be selected for the osteoconductive particles.

In some embodiments, the osteoconductive particles include or consist of synthetic polymers, bioactive glass, or bioceramics. In some embodiments, the osteoconductive particles include or consist of a silica-based bioglass. In some embodiments, the osteoconductive particles include or consist of a material that may be substantially crystalline, semi-crystalline, or amorphous. In some embodiments, the osteoconductive particles include or consist of one or more magnesium-containing compounds, for example, magnesium phosphate and magnesium sulfate. In some embodiments, the osteoconductive particles include or consist of calcium-containing compounds such as, for example, any of the calcium compounds discussed in U.S. Patent Application Publication No. US 2006/0008504 A1. More particularly, in certain embodiments, the osteoconductive particles include or consist of a calcium phosphate. In some embodiments, the osteoconductive particles include or consist of substantially crystalline calcium phosphate. In other embodiments, the osteoconductive particles include or consist of substantially amorphous calcium phosphate. In some embodiments, the calcium phosphate includes one or more of the following: monocalcium phosphate monohydrate, monocalcium phosphate anhydrous, dicalcium phosphate dihydrate, dicalcium phosphate anhydrous, tetracalcium phosphate, calcium orthophosphate phosphate, calcium pyrophosphate, α-tricalcium phosphate, β-tricalcium phosphate, and hydroxyapatites. In some embodiments, the osteoconductive particles include or consist of one or more calcium compounds having a chemical formula selected from the following: CaHPO₄·nH₂O, α-Ca₃(PO₄)₂, α-bar-Ca₃(PO₄)₂, β-Ca₃(PO₄)₂, Ca₅(PO₄)₃OH, Ca₁₀(PO₄)₆(OH)₂, Ca₄O(PO₄)₂, CaP₄O₁₁, Ca₂P₂O₇, Ca(H₂PO₄)₂·nH₂O, Ca₈H₂(PO₄)₆·nH₂O, or any combination thereof, where n is a number ranging from 0 to 5. In some embodiments, the osteoconductive particles include or consist essentially of hydroxyapatite. In some embodiments, the osteoconductive particles do not contain hydroxyapatite. In preferred embodiments, the osteoconductive particles include or consist of particles of β-tricalcium phosphate (β-Ca₃(PO₄)₂). In some embodiments, the osteoconductive particles include a calcium phosphate, a magnesium phosphate, a magnesium sulfate, a silica based bioglass, or mixtures thereof.

### II. Polymer Matrix

The osteoconductive particles discussed herein are dispersed within a three-dimensional polymer matrix according to the present invention. The osteoconductive particles in some embodiments may be distributed substantially evenly throughout the three dimensional polymer matrix. In some embodiments, the osteoconductive particles are randomly dispersed throughout the polymer matrix. The polymer matrix binds or adheres the osteoconductive particles together to form the composites of the present invention. Moreover, in some embodiments, the polymer matrix provides mechanical stability and other properties to composites of the present invention.

In some embodiments, the polymer matrix makes up about 5% to about 80% of the weight of the composite. In some embodiments, the polymer matrix makes up about 10% to about 50% of the weight of the composite. In some embodiments, the polymer matrix makes up about 20% to about 40% of the weight of the composite. In yet further embodiments, the polymer matrix makes up about 30% of the weight of the composite. In some embodiments, the polymer matrix makes up at least 10% to about 50% of the weight of the composite. In some embodiments, the polymer matrix makes up no more than 80% of the weight of the composite.

The polymer matrix in certain preferred embodiments includes or consists of a fully resorbable, biocompatible polymer material. In some embodiments, the polymer material is a synthetic polymer material. The polymer material is capable of binding to the osteoconductive particles and, in further embodiments, be capable of forming a rigid structure in combination with the osteoconductive particles at about normal human body temperature (e.g., about 37 degrees C). In some embodiments, the polymer material is selected to have a melting point above 37 degrees C. In some embodiments, the polymer material is selected to have a melting point less than 100 degrees C. In some embodiments, the polymer material is selected to have a melting point from about 40 degrees C to about 90 degrees C. In some embodiments, the polymer material is selected to have a melting point from about 50 degrees C to about 80 degrees C. In some embodiments, the polymer material is selected to have a melting point from about 55 degrees C to about 75 degrees C. In some embodiments, the polymer material is selected to have a melting point of about 60 degrees C.

In some embodiments, the polymer material by itself is not substantially osteoconductive. Yet, in some embodiments, the polymer material of the polymer matrix includes or consists of a biodegradable polyester. The polymer material includes or consists of a polycaprolactone in some embodiments. For example, the polymer material includes or consists of poly-(ε)-caprolactone. The polymer material may generally have the formula: 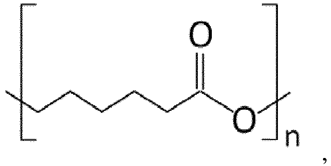 where n is equal to the number of repeat units. In some embodiments, the polymer material includes or consists essentially of poly-(ε)-caprolactone or another poly (hydroxy carboxylic acid) based polymer. In other embodiments, the polymer material includes or consists of one or more copolymers of caprolactone, including block and diblock copolymers. Non-limiting examples of such materials include copolymers of caprolactone with poly(lactic acid), glycolic acid, and/or poly(ethylene glycol), or combinations thereof. In some embodiments, the polymer material includes one or more materials selected from the following families: PLA-CL, PLA-GA-CL, PCL-PEG, or combinations thereof. In some embodiments, the polymer material includes or consists of, for example, one or more of poly(DL-lactide-co-caprolactone), poly(L-lactide-co-caprolactone-co-glycolide), poly(ε-caprolactone)-poly(ethylene glycol), and combinations thereof. In some embodiments, the polymer material includes or consists of poly(lactide-co-caprolactone). In other embodiments, the polymer material does not include polycaprolactone or caprolactone copolymers. In some embodiments, the polymer material does not include polycaprolactone or caprolactone copolymers but has one or more handling properties similar to or substantially the same as those of polycaprolactone. In further embodiments, the polymer material includes a biocompatible, resorbable polymer that, when in the presence of a plasticizer or other additive, has one or more handling properties similar to or substantially the same as those of polycaprolactone. Handling properties can include, for example, pliability, shapeability, strength, modulus, melting point/glass transition temperature, etc.

The polymer matrix is porous. For example, the polymer matrix in some implementations has a porosity of about 20% to about 80%, where the percent porosity refers to the volume of the pores as a percentage of the total volume of the polymer matrix. In some embodiments, the polymer matrix has a porosity of about 30% to about 70%. In some embodiments, the polymer matrix has a porosity of about 40% to about 60%. In some embodiments, the polymer matrix has a porosity of about 50%. The pores of the polymer matrix can be configured to define a plurality of fluid passageways that expose at least a portion of the osteoconductive particles dispersed within the polymer matrix to an exterior of the polymer matrix. These fluid passageways, according to some embodiments, permit blood and/or other body fluids to perfuse into the polymer matrix after implantation of the composite and come into direct contact with at least some of the osteoconductive particles. It is believed that permitting fluid communication between the surrounding tissue (e.g., bone tissue) and the osteoconductive particles within the polymer matrix according to these embodiments helps facilitate osteosynthesis and bone ingrowth. For example, this fluid communication may allow for the transport and supply of nutrients and biological factors that facilitates new bone growth. Moreover, the porosity of the polymer matrix in some embodiments allows for blood vessels to grow into the composite which also encourages new tissue development. Therefore, total encapsulation of the osteoconductive particles by the polymer material that prevents or inhibits fluid contact with the osteoconductive particles is avoided. At least a portion of the external surface of the osteoconductive particles is not directly in contact with the polymer material of the polymer matrix. In some embodiments, a majority of the external surface (e.g., greater than 50% of the external surface area) of the osteoconductive particles is not directly in contact with the polymer material of the polymer matrix.

The polymer matrix includes a plurality of polymer particles (e.g., polycaprolactone particles) that are affixed to external surfaces of the osteoconductive particles, the osteoconductive particles being physically connected to each other by the polymer particles. The polymer particles may, for example, be formed from one or more of the polymer materials described above and herein. A portion of the external surfaces of the osteoconductive particles are not in contact with or directly covered by the polymer particles so as not to inhibit fluid communication between the surrounding tissue and the osteoconductive particles. In some embodiments not recited in the claims, the polymer matrix includes agglomerated polymer particles (e.g., polycaprolactone particles) in a porous configuration with interposed osteoconductive particles within the polymer matrix such that at least a portion of the surface of the osteoconductive particles are exposed to pores within the matrix, the pores being interconnected to expose at least a portion of the osteoconductive particles to the outer surface of the composite. According to some further embodiments, the polymer particles create a form fit with the osteconductive particles when heated during the manufacturing methods (described further below) and maintain the form fit after cooling down.

The polymer particles according to these embodiments are smaller in size than the osteoconductive particles (e.g., in broadest dimension). For example, in some embodiments, the polymer matrix includes or consists of polymer particles from about 100 µm to about 355 µm in a broadest dimension (e.g., in diameter). In some embodiments, optimal polymer particle size and polymer particle distribution of the polymer matrix may be dependent from the particle size and particle size distribution of the osteconductive particles. In one embodiment, a composite according to the present invention includes polymer particles having a particle size ranging from about 100 µm to about 355 µm in a broadest dimension and osteoconductive particles having a particle size ranging from about 0.5 mm to about 0.7 mm in a broadest dimension.

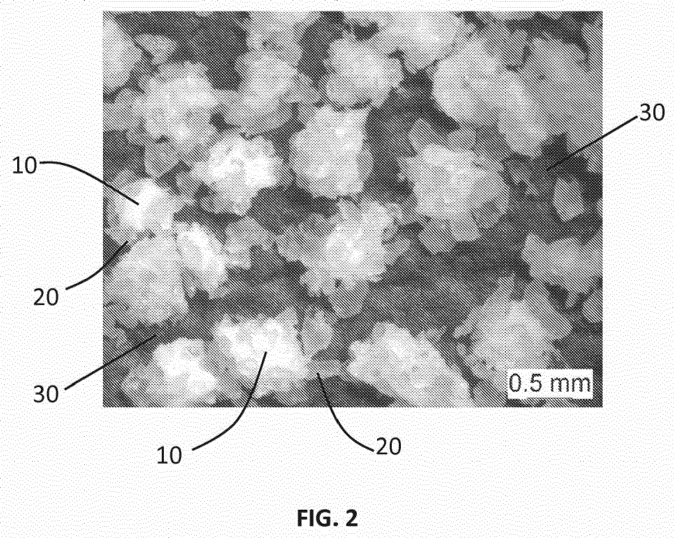

FIG. 2 shows a micrograph image of an example composite according to one embodiment of the invention. As can be seen in this image, the composite includes osteoconductive particles 10 which are each partially surrounded by smaller polymer particles 20. The composite further includes pore spaces 30 to which at least a portion of osteoconductive particles 10 are exposed. Pore spaces 30 define fluid passageways which can allow for the penetration of blood or other body fluids after implantation of the composite into a patient.

### III. Additives

The composites of the present invention further include one or more chemical additives incorporated within the polymer matrix. The chemical additive is selected to modify one or more properties of the polymer matrix. In some embodiments, the one or more additives include a plasticizer. In some embodiments, the one or more additives does not include a plasticizer. The chemical additive is configured to modify one or more of acidity, degradation rate, melting point, hydrophilicity, and hydrophobicity of the polymer matrix. In some embodiments, the chemical additive modifies the biocompatibility of the composite, for example, by preventing fibrous tissue growth or encapsulation. The chemical additive, in certain embodiments, is distinct from the polymer material of the polymer matrix and does not form a copolymer therewith. In some embodiments, the chemical additive is a synthetic chemical. In other embodiments, the chemical additive is not synthetic. In some embodiments, the chemical additive is a natural chemical and/or derived from natural sources.

In some embodiments, the additive is selected to increase the degradation rate of the polymer matrix and therefore expedites resorption of the composite after implantation. This is achieved, according to some embodiments, where the additive directly or indirectly facilitates the breaking of chemical bonds within the polymer material of the polymer matrix. In one exemplary embodiment, where the polymer matrix is made of polycaprolactone, the additive may be configured to create an acidic environment within the polymer matrix following implantation which accelerates the degradation of the polymer matrix by breaking the ester linkages of the polycaprolactone. Suitable additives according to this embodiment include biocompatible acidic compounds, which, for example, may be selected from the following: lactic acid, lactic acid dimer, lactic acid oligomer, capric acid monomer, capric acid dimer, capric acid oligomer, glycolic acid monomer, glycolic acid dimer, glycolic acid oligomer, ascorbic acid, citric acid, fatty acids and their metal salts, and combinations thereof. Preferably, the acidic compound is present in an amount sufficient to create a local acidic environment within the polymer matrix and composite without substantially impacting the natural pH level of the surrounding tissue following implantation.

In some embodiments, the one or more additives are selected to increase the hydrophilicity or the hydrophobicity of the composite. For example, in one embodiment, the additive may include a fatty acid as mentioned above or another substantially hydrophobic compound, which may in turn increase the hydrophobicity of the composite. In other embodiments, a hydrophilic compound may be included as the additive which may increase the hydrophilicity of the composite and/or the wettability of the polymer matrix. Increased wettability and/or hydrophilicity may be useful in some embodiments in order to facilitate perfusion of body fluids into the composite following implantation which could encourage osteosynthesis and/or resorption of the composite. In some embodiments, the increased wettability of the polymer matrix allows for an enhanced hydrophilic attack of the polymer matrix by the body fluids after implantation.

In some embodiments, the additive includes one or more polymers including, for example, a polymer selected from the following: polyethylene glycol (PEG), polyethylene oxide (PEO), polypropylene glycol (PPG), poly lactic acid (PLA), poly glycolic acid (PGA), and copolymers thereof. In some embodiments, the additive is a synthetic polymer. In some embodiments, the additive is a dimer or an oligomer. In some embodiments, the inclusion of one or more of these polymer additives to the composite modifies at least one of hydrophilicity, degradation profile (e.g., resorption rate), handling properties (e.g., pliability), and biocompatibility of the composite. In some embodiments, the additive is configured to modify one or more thermomechanical properties of the polymer matrix and the resulting composite. In some embodiments, the additive is configured to lower the melting point or glass transition temperature of the polymer matrix or composite such that the composite is capable of transitioning from the substantially rigid state to the substantially pliable state at a lower temperature.

### METHODS OF MANUFACTURE

Methods for making a composite according to some embodiments of the present invention include a) component preparation (e.g., preparing the osteoconductive particles and/or the polymer material), b) mixing of the components, and c) creation of a solid unit (e.g., the final composite). In some embodiments, two or more of the above steps may be combined in a single step. In some embodiments, for example, the polymer material can be prepared with processes using solvents (e.g., in a rotary evaporator or rotavapor) or using thermal processes (e.g., holt melt extrusion or heatable stirring unit). Example solvents include dioxan and ethylacetat. The polymer material can be either a single polymer or a combination of two or more different polymers with additives (e.g. citric acid, PLA dimer, oligomeric PLA, etc.) as described above.

Mixing the components (e.g. mixing the osteoconductive particles and the polymer material), according to some embodiments, can include for example, holt melt extrusion, solvent mixing, mixing in a heatable stirring unit, spray coating, dip coating generative matrix creation (e.g., 3D printing), and powder mixing at room temperature. A method, according to one embodiment, is to heat the polymer material in a first step up to about 140 degrees C (dependent on the polymer(s) used) and add osteconductive particles while stirring the mass.

Creation of a solid unit from the mixed components, in some embodiments, includes heating the mixture in a microwave or oven. In some embodiments, where a solvent is used, creation of a solid unit includes lyophilization or freeze drying process, and/or evaporation with a heating process (e.g. oven, vacuum oven). In further embodiments, creation of a solid unit includes a pressing or molding process to create a defined form and/or surface for the composite. In some embodiments, once the solid unit is created, it is allowed to cool (e.g., to about room temperature). The method may also include, in some embodiments, direct filling of the composite into functional packaging (e.g., a syringe, blister, or pouch).

A method for making a composite according to the present invention generally includes the steps of 1) mixing the osteoconductive particles with the polymer material of the polymer matrix and the chemical additive(s) to form a mixture, and 2) treating the mixture to bind the osteoconductive particles with the polymer material to create a solid unit, said polymer material forming the polymer matrix. In some embodiments, the method further includes shaping the mixture into a desired form. For example, the mixture in some embodiments is shaped in a mold or a press. In some embodiments, the mixture is shaped into a disc, cylinder, tablet, wafer, plug, cone, frustum, rod, or any other desired shape for the solid unit. In further embodiments, the solid unit is then packaged.

The mixing step includes mixing the osteoconductive particles with the polymer material at about room temperature. In other embodiments, the mixing step comprises mixing the osteoconductive particles with the polymer material in a heatable stirring unit. Moreover, in some embodiments, the polymer material is mixed with the chemical additive(s) prior to mixing with the osteoconductive particles. In further embodiments of the method, the mixing step includes coating the polymer material onto the osteoconductive particles. In some embodiments, coating the polymer material onto the osteoconductive particles includes spray coating and/or dip coating. In other embodiments, the polymer material and osteoconductive particles are hot melt extruded. The external surface of the osteoconductive particles is not entirely coated by the polymer material according to the invention.

After the mixture of osteoconductive particles, polymer material, and chemical additive(s) have been formed, in some embodiments the mixture is treated by heating the mixture. In some embodiments, heating the mixture includes heating the mixture to a temperature sufficient to cause the polymer material to bind to the osteoconductive particles. In some embodiments, heating the mixture includes exposing the mixture to microwave radiation.

In other embodiments, the mixing step includes combining the osteoconductive particles with the polymer material in a solvent, and the treating step includes removing the solvent from the mixture. In some embodiments, the solvent is configured to dissolve the polymer material but not the osteoconductive particles. In further embodiments, a dispersion of colloidal polymer particles suspended in the solvent is created. In some embodiments, removing the solvent includes, for example, lyophilization and/or evaporation of the solvent. In some embodiments, removal of the solvent is facilitated by the application of heat and/or a vaccuum.

### EXAMPLE 1

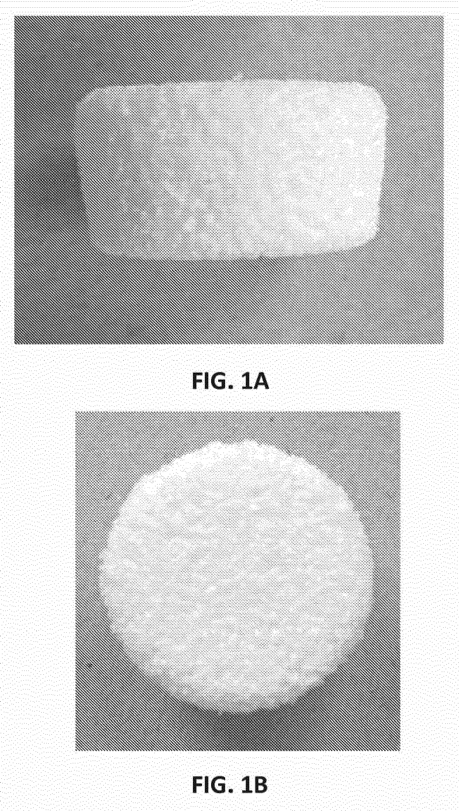

FIGS. 1A and 1B show an example composite including approximately 30% by weight polycaprolactone and 70% by weight of beta-tricalcium phosphate particles (0.5 mm to 0.7 mm in diameter). The composite was shaped into a generally round tab as shown.

### EXAMPLE 2

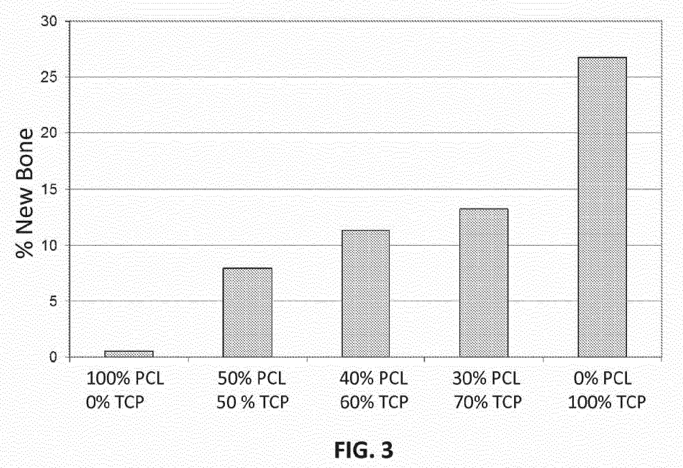

Composites including varying amounts of beta-tricalcium phosphate (TCP) particles and polycaprolactone (PCL) were prepared and implanted into sheep. The amounts tested included about 0% TCP/100% PCL, about 50% TCP/50% PCL, about 60% TCP/40% PCL, about 70% TCP/30% PCL, and about 100% TCP, where the stated percentages are by weight of the total composite. The percentage of new bone growth into each composite was measured after eight weeks and shown in the graph of FIG. 3. As can be seen, the amount of new bone growth increased with the percentage of TCP.

### EXAMPLE 3

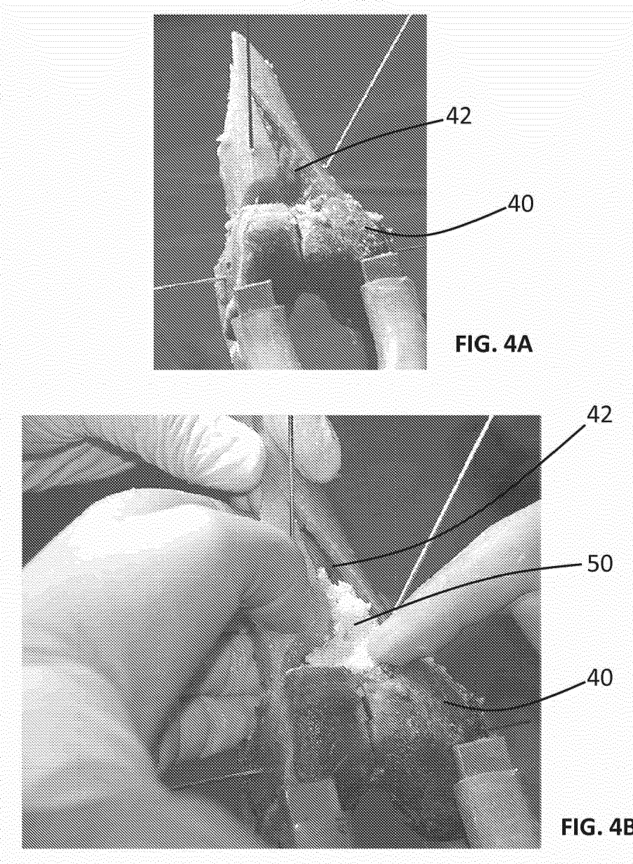

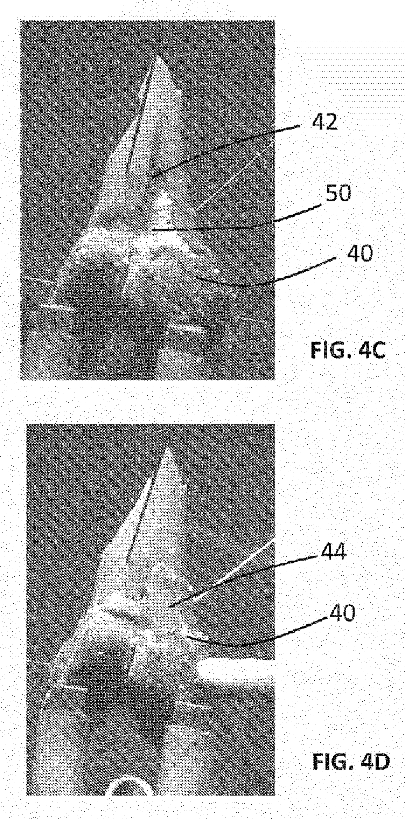

FIGS. 4A-4D are a series of photographs showing the implantation of an example composite 50 into a bone 40 in accordance with an embodiment of the present invention. In this example, bone 40 is a distal femur bone obtained from a cadaver having a cavity 42. As shown in FIGS. 4B and 4C, composite 50 was molded by hand into cavity 42 to at least partially fill cavity 42. Following implantation of composite 50, bone fragment 44 was repositioned over cavity 42 and composite 50.

### EXAMPLE 4

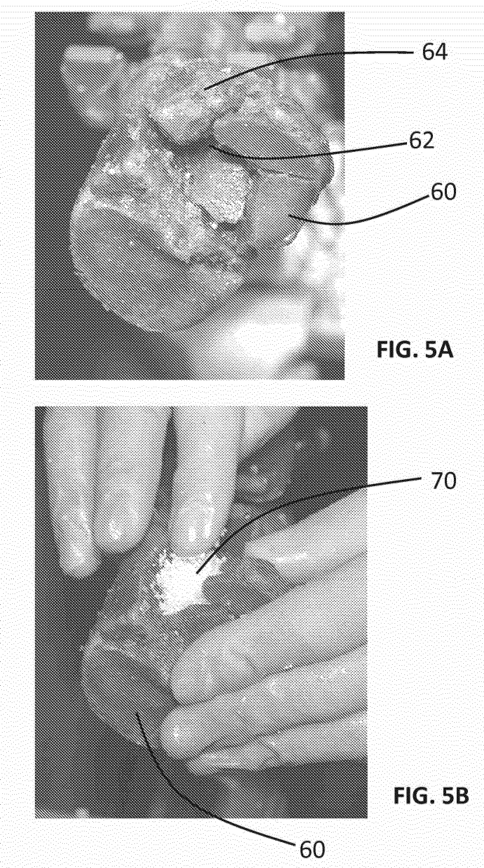

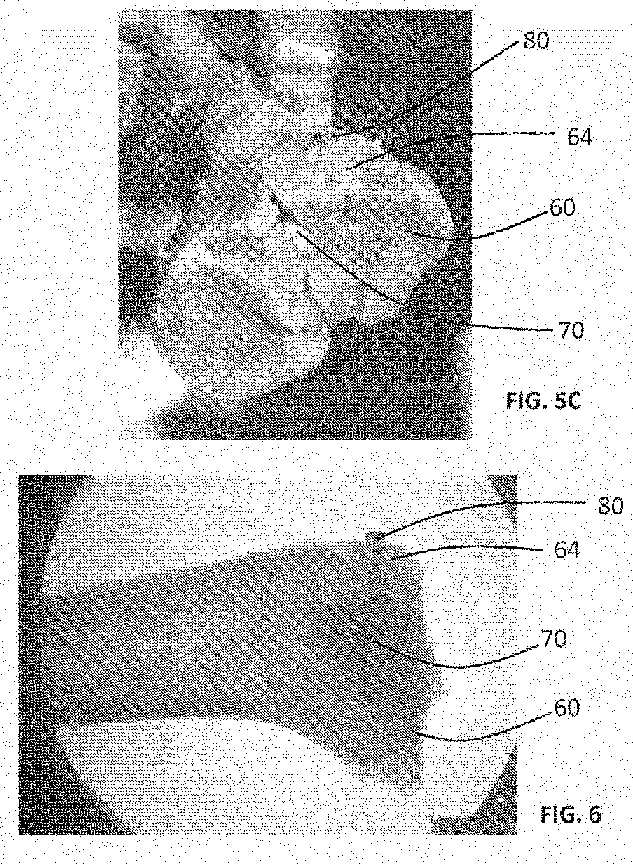

FIGS. 5A-5C are a series of photographs showing the implantation of a second example composite 70 into a bone 60 in accordance with a further embodiment of the present invention. In this example, bone 60 is a tibia bone obtained from a cadaver having a cavity 62 and bone fragment 64, as shown in FIG. 5A. Bone fragment 64 was removed to expose cavity 62 and composite 70 was molded by hand into cavity 62 to at least partially fill cavity 62 (FIG. 5B). After implantation of composite 70, bone fragment 64 was repositioned over cavity 62 and secured in place by screw 80, which was screwed into composite 70. FIG. 6 is a radiograph image of bone 60 showing screw 80 passing through bone fragment 64 and composite 70 (appearing as the darkened mass).

## Claims

1. A biocompatible, resorbable composite for osteosynthesis, the composite comprising:
osteoconductive particles dispersed within a porous polymer matrix, the polymer matrix comprising a plurality of fluid passageways that expose at least a portion of a plurality of the osteoconductive particles to an exterior of the polymer matrix, wherein the polymer matrix comprises polymer particles affixed to an external surface of the osteoconductive particles and a portion of the external surface of the osteoconductive particles is not covered by the polymer particles, wherein the polymer particles are smaller in broadest dimension than the osteoconductive particles, and wherein the osteoconductive particles and the polymer particles are aggregated to form the composite; and
a chemical additive incorporated within the polymer matrix, the chemical additive configured to modify one or more of acidity, degradation rate, melting point, hydrophilicity, and hydrophobicity of the polymer matrix.

2. The composite according to claim 1, wherein the polymer matrix comprises from 5% to 80% of the weight of the composite.

3. The composite according to preceding claim 2, wherein the polymer matrix comprises from 10% to 50% of the weight of the composite.

4. The composite according to any one of the preceding claims, wherein the osteoconductive particles comprise a calcium phosphate, a magnesium phosphate, a magnesium sulfate, a silica based bioglass, or mixtures thereof.

5. The composite according to any one of the preceding claims, wherein the osteoconductive particles consist essentially of beta-tricalcium phosphate or hydroxyapatite.

6. The composite according to any one of the preceding claims, wherein the polymer matrix consists essentially of poly-(ε)-caprolactone or another poly (hydroxy carboxylic acid) based polymer.

7. The composite according to claim 1, wherein the osteoconductive particles are physically connected by the polymer particles.

8. The composite according to any one of the preceding claims, wherein the polymer matrix has a porosity of 20% to 80%.

9. The composite according to any one of the preceding claims, wherein the osteoconductive particles each have a broadest dimension of 0.5 mm to 5.6 mm.

10. The composite according to any one of the preceding claims, wherein the chemical additive is configured to create an acidic environment within the polymer matrix when the composite is implanted.

11. The composite according to any one of the preceding claims, wherein the chemical additive is configured to increase the degradation rate of the polymer matrix when the composite is implanted.

12. The composite according to any one of the preceding claims, wherein the chemical additive is configured to facilitate breakage of ester linkages within the polymer matrix when the composite is implanted.

13. The composite according to any one of the preceding claims, wherein the chemical additive comprises an acidic compound.

14. The composite of claim 13, wherein the acidic compound is selected from the group consisting of lactic acid, lactic acid dimer, lactic acid oligomer, capric acid monomer, capric acid dimer, capric acid oligomer, glycolic acid monomer, glycolic acid dimer, glycolic acid oligomer, ascorbic acid, citric acid, fatty acids and their metal salts, and combinations thereof.

15. The composite according to any one of the preceding claims, wherein the chemical additive comprises a hydrophilic compound.

16. The composite according to any one of the preceding claims, wherein the chemical additive is configured to increase the wettability of the polymer matrix.

17. The composite according to any one of the preceding claims, wherein the chemical additive comprises a polymer.

18. The composite of claim 17, wherein the polymer is selected from the group consisting of polyethylene glycol, polyethylene oxide, polypropylene glycol, poly lactic acid, poly glycolic acid, and copolymers thereof.

19. The composite according to any one of the preceding claims, wherein the chemical additive is not copolymerized with the polymer matrix.

20. The composite according to any one of the preceding claims, wherein the composite is completely synthetic.

21. The composite according to any one of the preceding claims, wherein the polymer matrix comprises polycaprolactone and/or a caprolactone copolymer.

22. The composite according to any one of the preceding claims, wherein the chemical additive comprises a plasticizer, and wherein the polymer matrix comprises a material with handling properties similar to polycaprolactone in the presence of said plasticizer.

23. The composite according to any one of the preceding claims, wherein the osteoconductive particles comprise a ceramic material.

24. The composite according to any one of the preceding claims, wherein the osteoconductive particles comprise crystalline, semi-crystalline, or amorphous calcium phosphate.

25. The composite according to any one of the preceding claims, wherein the osteoconductive particles comprise a bioactive glass.

26. A method for making the composite according to any one of the preceding claims, said method comprising the steps of:
mixing the osteoconductive particles with polymer material and the chemical additive to form a mixture; and
treating the mixture to bind the osteoconductive particles with the polymer material to create a solid unit, said polymer material forming the polymer matrix;
wherein the mixing step comprises mixing the osteoconductive particles with polymer material particles at about room temperature.

27. The method of claim 26, wherein the mixing step comprises combining the osteoconductive particles with the polymer material in a solvent, and wherein the treating step comprises removing the solvent.

28. The method of claim 27, wherein removing the solvent includes lyophilization and/or evaporation of the solvent.

29. The method of claim 26, wherein the mixing step comprises coating the polymer material onto the osteoconductive particles.

30. The method of claim 26, wherein coating the polymer material onto the osteoconductive particles includes spray coating and/or dip coating.

31. The method of claim 26, wherein the treating step comprises heating the mixture.

32. The method of claim 26, wherein the treating step comprises exposing the mixture to microwave radiation.

33. The method of claim 26, wherein the mixing step comprises mixing the osteoconductive particles with the polymer material with a heatable stirring unit.

34. The method according to any one of claims 26-33, wherein the polymer material is mixed with the chemical additive prior to mixing with the osteoconductive particles.

35. The method according to any one of claims 26-34, further comprising shaping the mixture using a press or mold.

36. The method according to claim 35, wherein the mixture is shaped into a disc, cylinder, tablet, wafer, plug, cone, frustum, or rod.

37. The method according to any one of claims 26-36, further comprising packaging the solid unit.

## Patentansprüche

1. Biokompatibler, resorbierbarer Verbundstoff für die Osteosynthese, wobei der Verbundstoff umfasst:
in einer porösen Polymermatrix dispergierte osteokonduktive Teilchen, wobei die Polymermatrix eine Vielzahl von Fluiddurchgängen umfasst, die mindestens einen Teil einer Vielzahl der osteokonduktiven Teilchen einer Außenseite der Polymermatrix aussetzen, wobei die Polymermatrix Polymerteilchen umfasst, die an der Außenseite der osteokonduktiven Teilchen befestigt sind, und ein Teil der Außenseite der osteokonduktiven Teilchen nicht durch die Polymerteilchen bedeckt ist, wobei die Polymerteilchen in der breitesten Dimension kleiner sind als die osteokonduktiven Teilchen und wobei die osteokonduktiven Teilchen und die Polymerteilchen derart aggregiert sind, dass der Verbundstoff erhalten wird; und
ein chemisches Additiv, das in die Polymermatrix eingebaut ist, wobei das chemische Additiv so ausgelegt ist, dass es eine oder mehrere der Eigenschaften Azidität, Abbaurate, Schmelzpunkt, Hydrophilie und Hydrophobie der Polymermatrix modifiziert.

2. Verbundstoff nach Anspruch 1, wobei die Polymermatrix 5% bis 80%, bezogen auf das Gewicht des Verbundstoffs, ausmacht.

3. Verbundstoff nach dem vorhergehenden Anspruch 2, wobei die Polymermatrix 10% bis 50%, bezogen auf das Gewicht des Verbundstoffs, ausmacht.

4. Verbundstoff nach einem der vorhergehenden Ansprüche, wobei die osteokonduktiven Teilchen ein Calciumphosphat, ein Magnesiumphosphat, ein Magnesiumsulfat, ein auf Siliciumdioxid basierendes Bioglas oder Gemische davon umfassen.

5. Verbundstoff nach einem der vorhergehenden Ansprüche, wobei die osteokonduktiven Teilchen im Wesentlichen aus Beta-Tricalciumphosphat oder Hydroxyapatit bestehen.

6. Verbundstoff nach einem der vorhergehenden Ansprüche, wobei die Polymermatrix im Wesentlichen aus Poly-(ε)-caprolacton oder einem anderen Polymer auf Poly(hydroxycarbonsäure)-Basis besteht.

7. Verbundstoff nach Anspruch 1, wobei die osteokonduktiven Teilchen durch die Polymerteilchen physikalisch verbunden sind.

8. Verbundstoff nach einem der vorhergehenden Ansprüche, wobei die Polymermatrix eine Porosität von 20% bis 80% aufweist.

9. Verbundstoff nach einem der vorhergehenden Ansprüche, wobei die osteokonduktiven Teilchen jeweils eine breiteste Abmessung von 0,5 mm bis 5,6 mm haben.

10. Verbundstoff nach einem der vorhergehenden Ansprüche, wobei das chemische Additiv so ausgelegt ist, dass es eine saure Umgebung in der Polymermatrix erzeugt, wenn der Verbundstoff implantiert wird.

11. Verbundstoff nach einem der vorhergehenden Ansprüche, wobei das chemische Additiv so ausgelegt ist, dass es die Abbaurate der Polymermatrix erhöht, wenn der Verbundstoff implantiert wird.

12. Verbundstoff nach einem der vorhergehenden Ansprüche, wobei das chemische Additiv so ausgelegt ist, dass es das Brechen von Esterbindungen in der Polymermatrix erleichtert, wenn der Verbundstoff implantiert wird.

13. Verbundstoff nach einem der vorhergehenden Ansprüche, wobei das chemische Additiv eine saure Verbindung umfasst.

14. Verbundstoff nach Anspruch 13, wobei die saure Verbindung ausgewählt ist aus der Gruppe bestehend aus Milchsäure, Milchsäuredimer, Milchsäureoligomer, Caprinsäuremonomer, Caprinsäuredimer, Caprinsäureoligomer, Glycolinsäuremonomer, Glycolinsäuredimer, Glycolsäureoligomer, Ascorbinsäure, Zitronensäure, Fettsäuren und deren Metallsalzen und Kombinationen davon.

15. Verbundstoff nach einem der vorhergehenden Ansprüche, wobei das chemische Additiv eine hydrophile Verbindung umfasst.

16. Verbundstoff nach einem der vorhergehenden Ansprüche, wobei das chemische Additiv so ausgelegt ist, dass es die Benetzbarkeit der Polymermatrix erhöht.

17. Verbundstoff nach einem der vorhergehenden Ansprüche, wobei das chemische Additiv ein Polymer umfasst.

18. Verbundstoff nach Anspruch 17, wobei das Polymer ausgewählt ist aus der Gruppe bestehend aus Polyethylenglykol, Polyethylenoxid, Polypropylenglykol, Polymilchsäure, Polyglykolsäure und Copolymeren davon.

19. Verbundstoff nach einem der vorhergehenden Ansprüche, wobei das chemische Additiv nicht mit der Polymermatrix copolymerisiert ist.

20. Verbundstoff nach einem der vorhergehenden Ansprüche, wobei der Verbundstoff vollständig synthetisch ist.

21. Verbundstoff nach einem der vorhergehenden Ansprüche, wobei die Polymermatrix Polycaprolacton und/oder ein Caprolacton-Copolymer umfasst.

22. Verbundstoff nach einem der vorhergehenden Ansprüche, wobei das chemische Additiv einen Weichmacher umfasst, und wobei die Polymermatrix ein Material mit Handhabungseigenschaften ähnlich zu Polycaprolacton in Gegenwart des Weichmachers umfasst.

23. Verbundstoff nach einem der vorhergehenden Ansprüche, wobei die osteokonduktiven Teilchen ein keramisches Material umfassen.

24. Verbundstoff nach einem der vorhergehenden Ansprüche, wobei die osteokonduktiven Teilchen kristallines, semikristallines oder amorphes Calciumphosphat umfassen.

25. Verbundstoff nach einem der vorhergehenden Ansprüche, wobei die osteokonduktiven Teilchen ein bioaktives Glas umfassen.

26. Verfahren zur Herstellung des Verbundstoffs nach einem der vorhergehenden Ansprüche, wobei das Verfahren die folgenden Schritte umfasst:
Mischen der osteokonduktiven Teilchen mit Polymermaterial und dem chemischen Zusatzstoff, um ein Gemisch zu bilden; und
Behandeln des Gemischs, um die osteokonduktiven Teilchen mit dem Polymermaterial zu verbinden, um eine feste Einheit zu erzeugen, wobei das Polymermaterial die Polymermatrix bildet;
wobei der Mischschritt das Mischen der osteokonduktiven Teilchen mit Polymermaterialteilchen bei etwa Raumtemperatur umfasst.

27. Verfahren nach Anspruch 26, wobei der Mischschritt das Kombinieren der osteokonduktiven Teilchen mit dem Polymermaterial in einem Lösungsmittel umfasst, und wobei der Behandlungsschritt das Entfernen des Lösungsmittels umfasst.

28. Verfahren nach Anspruch 27, wobei das Entfernen des Lösungsmittels das Lyophilisieren und/oder Verdampfen des Lösungsmittels einschließt.

29. Verfahren nach Anspruch 26, wobei der Mischschritt das Aufbringen des Polymermaterials auf die osteokonduktiven Teilchen umfasst.

30. Verfahren nach Anspruch 26, wobei das Aufbringen des Polymermaterials auf die osteokonduktiven Teilchen Sprühbeschichten und/oder Tauchbeschichten einschließt.

31. Verfahren nach Anspruch 26, wobei der Behandlungsschritt das Erhitzen des Gemischs umfasst.

32. Verfahren nach Anspruch 26, wobei der Behandlungsschritt das Aussetzen des Gemischs gegenüber Mikrowellenstrahlung umfasst.

33. Verfahren nach Anspruch 26, wobei der Mischschritt das Mischen der osteokonduktiven Teilchen mit dem Polymermaterial mit einer heizbaren Rühreinheit umfasst.

34. Verfahren nach einem der Ansprüche 26 bis 33, wobei das Polymermaterial vor dem Mischen mit den osteokonduktiven Teilchen mit dem chemischen Additiv gemischt wird.

35. Verfahren nach einem der Ansprüche 26 bis 34, zudem umfassend das Formen des Gemischs unter Verwendung einer Presse oder einer Form.

36. Verfahren nach Anspruch 35, wobei das Gemisch zu einer Scheibe, einem Zylinder, einer Tafel, einem Wafer, einem Stopfen, einem Kegel, einem Kegelstumpf oder einer Stange geformt wird.

37. Verfahren nach einem der Ansprüche 26 bis 36, zudem umfassend das Verpacken der festen Einheit.

## Revendications

1. Composite résorbable, biocompatible pour ostéosynthèse, le composite comprenant :
des particules ostéoconductrices dispersées dans une matrice polymère poreuse, la matrice polymère comprenant une pluralité de passages de fluide exposant au moins une partie de la pluralité de particules ostéoconductrices vers l'extérieur de la matrice polymère, la matrice polymère comprenant des particules polymères fixées sur une surface externe des particules ostéoconductrices et une partie de la surface externe des particules ostéoconductrices n'étant pas recouverte par les particules polymères, les particules polymères étant plus petites dans leur dimension la plus large que les particules ostéoconductrices et les particules ostéoconductrices et les particules polymères étant agglomérées pour former le composite ; et
un additif chimique incorporé dans la matrice polymère, l'additif chimique étant conçu pour modifier l'un ou plusieurs parmi l'acidité, la vitesse de dégradation, le point de fusion, l'hydrophilicité et l'hydrophobicité de la matrice polymère.

2. Composite selon la revendication 1, la matrice polymère représentant 5% jusqu'à 80% du poids du composite.

3. Composite selon la revendication 2, la matrice polymère représentant 10% jusqu'à 50% du poids du composite.

4. Composite selon l'une quelconque des revendications précédentes, les particules ostéoconductrices comprenant un phosphate de calcium, un phosphate de magnésium, un sulfate de magnésium, un bioverre à base de silice ou des mélanges correspondants.

5. Composite selon l'une quelconque des revendications précédentes, les particules ostéoconductrices étant essentiellement constituées de bêta-phosphate tricalcique ou d'hydroxyapatite.

6. Composite selon l'une quelconque des revendications précédentes, la matrice polymère étant essentiellement constituée de poly(ε-caprolactone) ou d'un autre polymère à base de poly(acide hydroxycarboxylique).

7. Composite selon la revendication 1, les particules ostéoconductrices étant reliées physiquement par les particules polymères.

8. Composite selon l'une quelconque des revendications précédentes, la matrice polymère présentant une porosité de 20% jusqu'à 80%.

9. Composite selon l'une quelconque des revendications précédentes, les particules ostéoconductrices ayant chacune une dimension la plus large de 0,5 mm jusqu'à 5,6 mm.

10. Composite selon l'une quelconque des revendications précédentes, l'additif chimique étant conçu pour créer un environnement acide dans la matrice polymère lorsque le composite est implanté.

11. Composite selon l'une quelconque des revendications précédentes, l'additif chimique étant conçu pour augmenter la vitesse de dégradation de la matrice polymère lorsque le composite est implanté.

12. Composite selon l'une quelconque des revendications précédentes, l'additif chimique étant conçu pour faciliter la rupture de liaisons ester dans la matrice polymère lorsque le composite est implanté.

13. Composite selon l'une quelconque des revendications précédentes, l'additif chimique comprenant un composé acide.

14. Composite selon la revendication 13, le composé acide étant choisi dans le groupe constitué par l'acide lactique, un dimère d'acide lactique, un oligomère d'acide lactique, un monomère d'acide caprique, un dimère d'acide caprique, un oligomère d'acide caprique, un monomère d'acide glycolique, un dimère d'acide glycolique, un oligomère d'acide glycolique, l'acide ascorbique, l'acide citrique, les acides gras et leurs sels métalliques et les combinaisons correspondantes.

15. Composite selon l'une quelconque des revendications précédentes, l'additif chimique comprenant un composé hydrophile.

16. Composite selon l'une quelconque des revendications précédentes, l'additif chimique étant conçu pour augmenter l'aptitude au mouillage de la matrice polymère.

17. Composite selon l'une quelconque des revendications précédentes, l'additif chimique comprenant un polymère.

18. Composite selon la revendication 17, le polymère étant choisi dans le groupe constitué par le polyéthylèneglycol, le poly(oxyde d'éthylène), le polypropylèneglycol, le poly(acide lactique), le poly(acide glycolique) et les copolymères correspondants.

19. Composite selon l'une quelconque des revendications précédentes, l'additif chimique n'étant pas copolymérisé avec la matrice polymère.

20. Composite selon l'une quelconque des revendications précédentes, le composite étant complètement synthétique.

21. Composite selon l'une quelconque des revendications précédentes, la matrice polymère comprenant une polycaprolactone et/ou un copolymère de caprolactone.

22. Composite selon l'une quelconque des revendications précédentes, l'additif chimique comprenant un plastifiant et la matrice polymère comprenant un matériau présentant des propriétés de manipulation similaires à celles de la polycaprolactone en présence dudit plastifiant.

23. Composite selon l'une quelconque des revendications précédentes, les particules ostéoconductrices comprenant un matériau en céramique.

24. Composite selon l'une quelconque des revendications précédentes, les particules ostéoconductrices comprenant du phosphate de calcium cristallin, du phosphate de calcium semi-cristallin ou du phosphate de calcium amorphe.

25. Composite selon l'une quelconque des revendications précédentes, les particules ostéoconductrices comprenant un verre bioactif.

26. Procédé de fabrication du composite selon l'une quelconque des revendications précédentes, ledit procédé comprenant les étapes de :
mélange des particules ostéoconductrices avec le matériau polymère et l'additif chimique pour former un mélange ; et
traitement du mélange pour lier les particules ostéoconductrices avec le matériau polymère afin de créer une unité solide, ledit matériau polymère formant la matrice polymère ;
l'étape de mélange comprenant le mélange des particules ostéoconductrices avec les particules de matériau polymère approximativement à température ambiante.

27. Procédé selon la revendication 26, l'étape de mélange comprenant la combinaison des particules ostéoconductrices avec le matériau polymère dans un solvant et l'étape de traitement comprenant l'élimination du solvant.

28. Procédé selon la revendication 27, l'élimination du solvant comprenant la lyophilisation et/ou l'évaporation du solvant.

29. Procédé selon la revendication 26, l'étape de mélange comprenant le dépôt du matériau polymère sur les particules ostéoconductrices.

30. Procédé selon la revendication 26, le dépôt du matériau polymère sur les particules ostéoconductrices comprenant le dépôt par pulvérisation et/ou le dépôt par trempage.

31. Procédé selon la revendication 26, l'étape de traitement comprenant le chauffage du mélange.

32. Procédé selon la revendication 26, l'étape de traitement comprenant l'exposition du mélange à un rayonnement microonde.

33. Procédé selon la revendication 26, l'étape de mélange comprenant le mélange des particules ostéoconductrices avec le matériau polymère au moyen d'une unité d'agitation pouvant être chauffée.

34. Procédé selon l'une quelconque des revendications 26-33, le matériau polymère étant mélangé avec l'additif chimique avant le mélange avec les particules ostéoconductrices.

35. Procédé selon l'une quelconque des revendications 26-34, comprenant en outre le façonnage du mélange à l'aide d'une presse ou d'un moule.

36. Procédé selon la revendication 35, le mélange étant façonné en un disque, un cylindre, une pastille, une plaquette, un bouchon, un cône, un tronc de cône ou une tige.

37. Procédé selon l'une quelconque des revendications 26-36, comprenant en outre l'emballage de l'unité solide.
